# EUROPEAN PATENT SPECIFICATION

(11) **EP 0 755 387 B1**
(45) Date of publication and mention of the grant of the patent: **04.08.1999**
(21) Application number: 95915472.5
(22) Date of filing: 30.03.1995
(51) Int. Cl.: C07D 305/14, C07B 63/02, C07C 249/16

(54) **OXIDATIVE PURIFICATION OF BIOMASS EXTRACTS CONTAINING TAXOL AND CEPHALOMANNINE USING OZONE AND GIRARD REAGENTS**
OXYDATIV REINIGUNG VON TAXOL UND CEPHALOMANNINE ENTHALTENDE BIOMASSEAUSZÜGE MIT HILFE VON OZON UND GIRARD REAGENS
PURIFICATION PAR OXYDATION D'EXTRAITS DE BIOMASSE CONTENANT DU TAXOL ET DE LA CEPHALOMANINE, A L'AIDE D'OZONE ET DE REACTIFS DE GIRARD

(30) Priority: 08.04.1994 US 224758
(43) Date of publication of application: 29.01.1997
(73) Proprietor: HAUSER CHEMICAL RESEARCH, INC., Boulder, CO 80301 (US)
(72) Inventor: MURRAY, Christopher, K., Boulder, CO 80301 (US); BECKVERMIT, Jeffrey, T., Boulder, CO 80301 (US); ANZIANO, Dominick, J., Boulder, CO 80301 (US)
(74) Representative: Baillie, Iain Cameron
(86) International application number: PCT/US95/03975
(87) International publication number: WO 95/27707

(56) References cited:
- US-A- 5 334 732
- : "", NATURE, , 1954, vol. 173, no. , pages 401 to 402
- NATURE, 1954, vol. 173, pages 401 to 402

## Description

### Technical Field

This invention relates to the separation of cephalomannine and related ozone oxidizable compounds from taxol and other taxanes in extracts of biomass containing a complex mixture of compounds. More particularly, this invention relates to a process to simplify the isolation and purification of taxol.

### Background Art

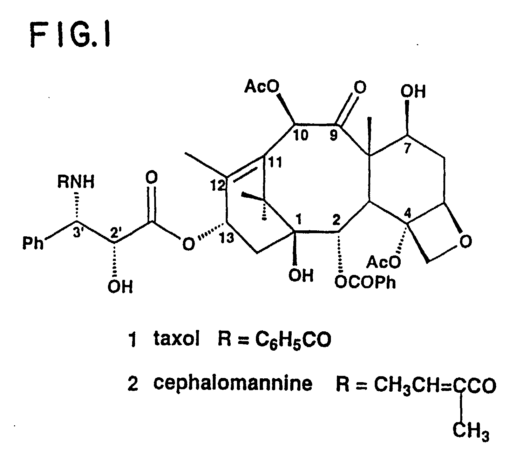

Extracts of Taxus species and cell cultures of Taxus contain taxol, a promising chemotherapeutic agent which was recently approved by the Food and Drug Administration for the treatment of refractory ovarian cancer. Currently, taxol is isolated on a large scale only from the bark of Taxus brevifolia. However, taxol can be potentially isolated on a large commercial scale from other parts of Taxus and also from cell culture of Taxus. The method of isolation of taxol from any natural source is complex. A particularly difficult and expensive part of the isolation of taxol is the separation of the closely-related diterpenoid compound cephalomannine. The structures and chemical properties of taxol and cephalomannine are similar. The only structural difference between the two compounds involves the side chain portion of the molecule (see **1** and **2** in Figure 1). Separation of taxol from cephalomannine is very difficult, but it can be done by various means of chromatography. This is an expensive process when performed on commercial scale.

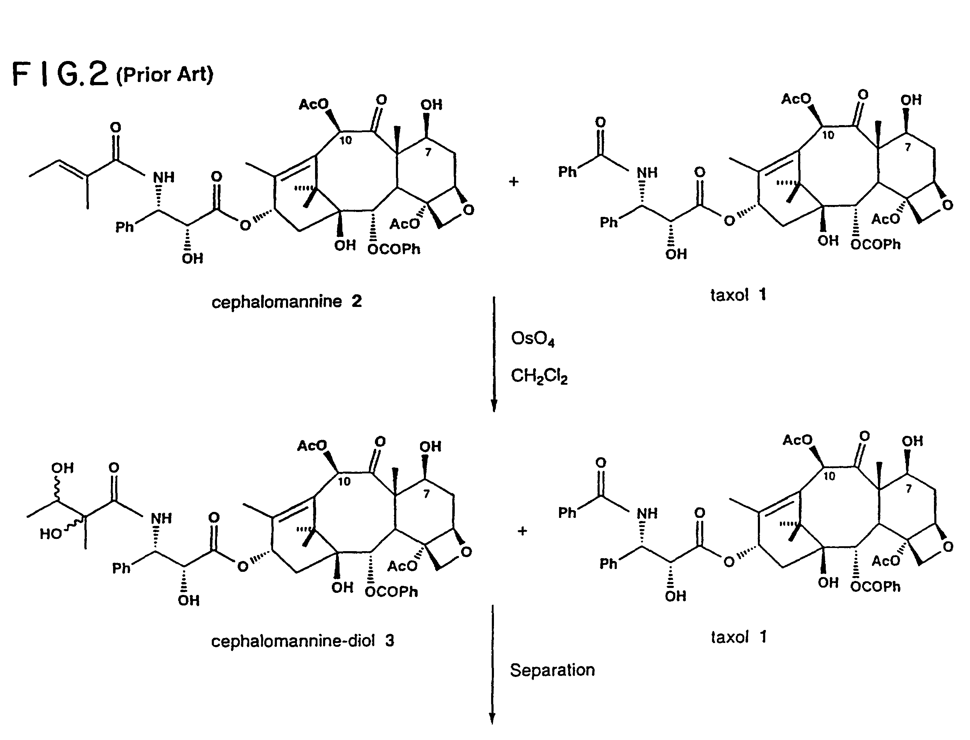

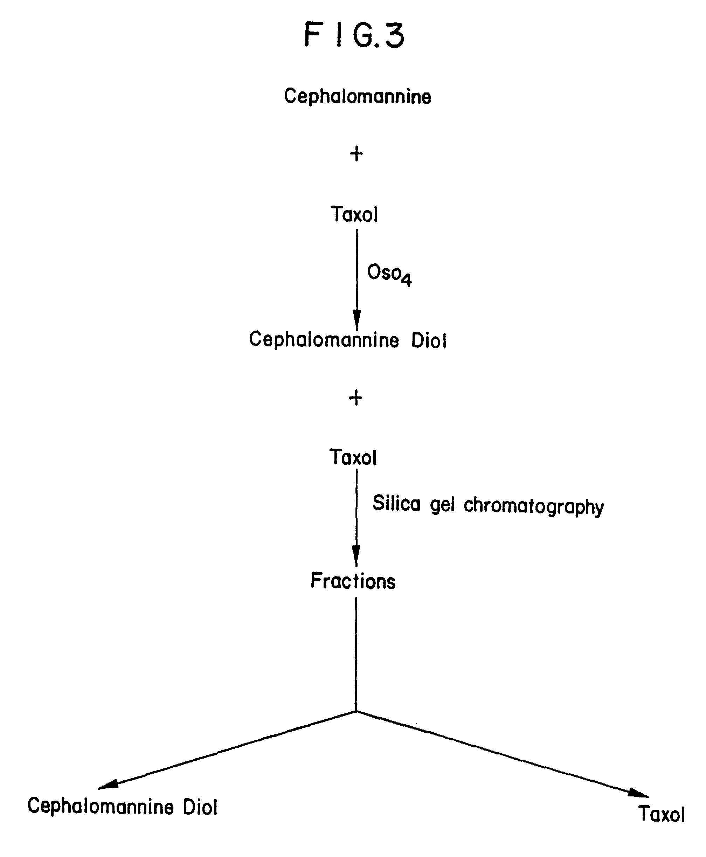

A method to modify the side chain of cephalomannine in the presence of taxol in purified and/or partially-purified taxane mixtures has been described by Kingston, Journal of Natural Products, Vol. 55, No. 2, pp. 259-261, February, 1992, incorporated herein by reference. In the partially-purified mixture taxol and cephalomannine were present in a total amount of about 80% by weight. Selective oxidation of the tiglate group of cephalomannine using osmium tetroxide under stoichiometric or catalytic conditions (Kingston's method) yielded a diol (compound 3), and did not affect the structure of taxol. The diol was separated from the reaction mixture by silica gel chromatography as shown in Figures 2 and 3 herein.

As explained in more detail hereinafter, the Kingston process is not suitable for use with unpurified taxane mixtures. As demonstrated in our laboratories, osmium tetroxide catalyzed oxidation of cephalomannine is not amenable to impure extracts from biomass that contain taxol because of the strict reaction conditions required (purity of substrates, anhydrous reagents, solvents, etc.) for good transformation. The use of osmium tetroxide in the bulk manufacture of a pharmaceutical is also undesirable due to the severe toxicity of osmium tetroxide and other osmium containing compounds.

There has not heretofore been provided a process for the inexpensive, simple, safe and effective separation of cephalomannine from taxol at a wide range of purities.

U.S. Patent No. 5,334,732 describes a process for oxidizing cephalomannine and related compounds in the presence of taxol. The process involves the use of ozone to oxidize cephalomannine and related easily oxidizable compounds while leaving taxol essentially unchanged. Taxol can then be separated from the oxidized cephalomannine and other oxidized compounds using conventional procedures such as chromatography.

### Disclosure of the Invention

In accordance with the present invention there is provided a process for separating cephalomannine and related compounds from taxol and other unreactive compounds. The process of the invention can be performed in a manner such that no significant loss of taxol results.

In accordance with the present invention there is provided a process for separating cephalomannine from a mixture containing cephalomannine and taxol in a solution, said process comprising the steps of: (a) contacting said mixture with ozone thereby oxidizing cephalomannine to form oxidized cephalomannine; (b) converting said oxidized cephalomannine to a water-soluble hydrazone; and (c) separating said water-soluble hydrazone from said mixture, wherein said mixture is prepared by dissolving a dried biomass extract into a solvent selected from the group consisting of hydrocarbons, halogenated solvents, alcohols, ethers, aldehydes, ketones, amines, organic acids, organic acid derivatives, esters, water and mixtures thereof.

The process of the invention is effective for mixtures of pure taxol, cephalomannine and other taxanes or for crude extracts of biomass containing very small amounts of these compounds along with other extractable compounds.

In the process of the invention, cephalomannine and other related compounds are chemically modified in the presence of taxol and other unreactive compounds by treating the mixture of these compounds with ozone. Ozone reacts with cephalomannine and related compounds to produce oxidized compounds, leaving essentially all of the taxol unchanged. The naturally occurring and oxidized compounds that contain reactive carbonyl functional groups are selectively converted to water soluble hydrazones and then separated from taxol and other taxanes by several methods that do not require expensive and inefficient chromatography steps.

As described herein, the process involves separation of three types of compounds; (a) taxol and other unreactive compounds, are separated from (b) cephalomannine and related compounds, and/or (c) other naturally occurring compounds that contain reactive carbonyl groups.

As used herein, the term "other unreactive compounds" includes taxanes and compounds that are not easily oxidized by ozone in the process of the present invention, and are not reactive to hydrazides or hydrazines in the process of the present invention.

As used herein, the term "related compounds" in regards to the process of the present invention, includes taxanes and other compounds that contain easily ozone oxidizable olefin functional groups, e.g., an olefin group that is not substituted on one end of the double bond. Olefins that are surprisingly not reactive in the process of the present invention include those with four bulky substituents (see Figure 1, carbons 11 and 12, for an example).

As used herein, the term "reactive carbonyl compounds" includes ketones and aldehydes that are reactive to hydrazides and hydrazines in the process of the present invention. By definition, reactive carbonyl compounds will be separated from taxol and other unreactive taxanes in mixtures whether or not cephalomannine is present in the mixture.

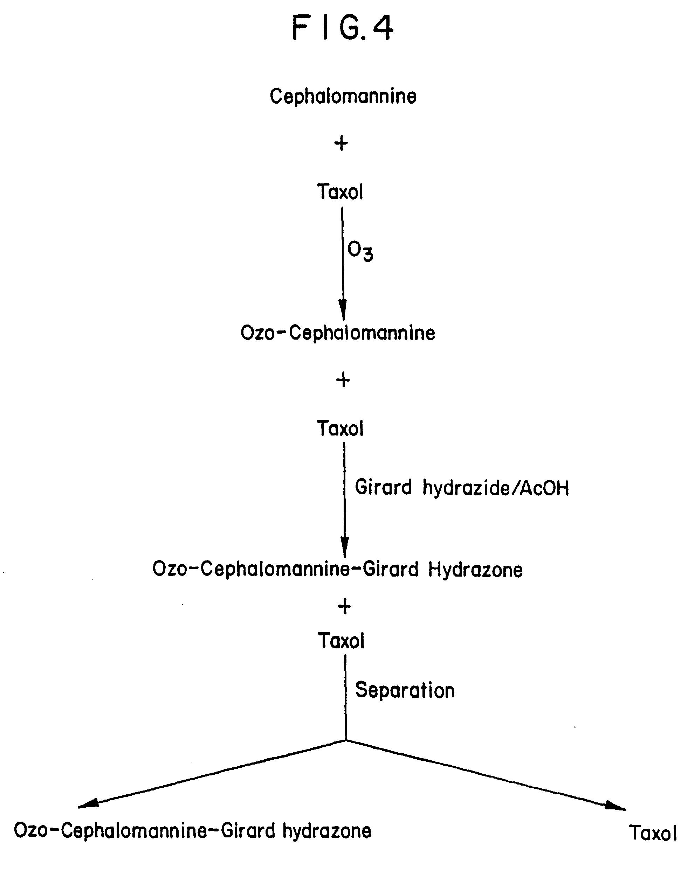

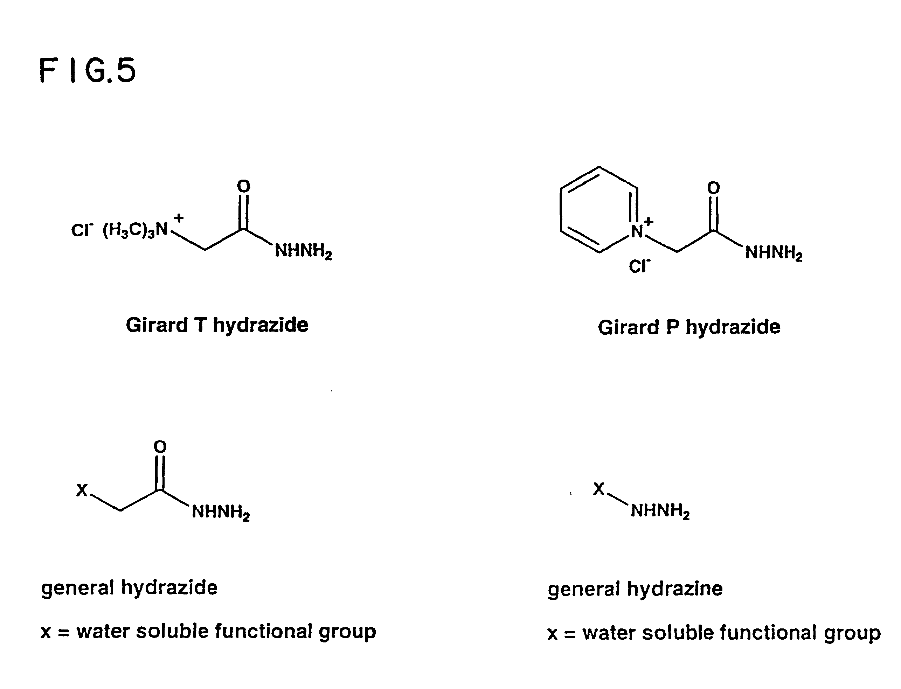

The process for separation of cephalomannine and related compounds from taxol and other unreactive compounds requires three steps. The first step is ozone oxidation, requiring only the solution of material (whatever the purity of taxol, cephalomannine and other taxanes) and an ozone generator capable of producing 1 to 10% ozone in an oxygen or air stream. The ozone is bubbled through the liquid mixture for the time required, and then the mixture is purged with an inert gas. The second step is conversion of the oxidized compounds which contain reactive ketone or aldehyde functional groups, including the oxidized cephalomannine, to water soluble hydrazones. The preferred water soluble hydrazones are the Girard hydrazones and have been used for other separations including the separation of keto-steroids from non-keto-steroids as described by D. A. Forsss, Nature, Vol. 173, pp. 401-402, 1954, incorporated herein by reference. The use of Girard reagents has not been reported previously for the isolation and purification of taxanes. The third step is the separation of the water soluble hydrazones of the oxidized cephalomannine and related compounds from taxol and other taxanes based on the differing solubilities and polarities of these materials. Two specific methods are reported herein for the separation which do not require inefficient chromatography. The three steps of the present process are summarized in Figure 4. The structures of the two most commonly used Girard reagents are shown in Figure 5.

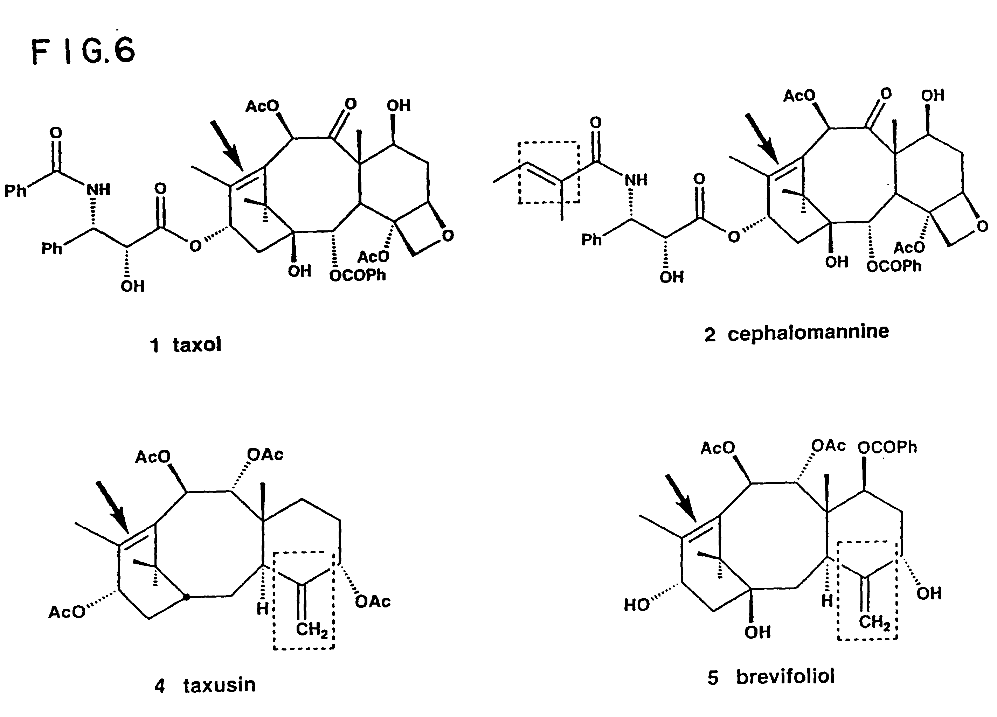

The ozonolysis process for selective oxidation of cephalomannine and related compounds in the presence of taxol and other taxanes leads to many new oxidized compounds in partially pure and impure mixtures of taxanes. Taxusin **4** and brevifoliol **5** (see Figure 6) are two compounds, for example, commonly found in Taxus brevifolia biomass that will most likely be oxidized along with cephalomannine. The oxidation will occur preferentially at the olefin functional groups outlined by dotted lines in Figure 6. The tetrasubstituted olefin indicated by the bold arrow in each of the structures in Figure 6 is much less reactive than are the less substituted olefins. Under conditions outlined herein, the tetrasubstituted olefin is essentially unchanged during the present oxidation process. Other ozone oxidation susceptible taxanes have functional groups similar to those outlined by the dotted lines in Figure 6. The high selectivity of the ozone oxidation process for specific taxanes in impure and partially pure mixtures is unexpected and can greatly simplify the purification process of taxol as described herein.

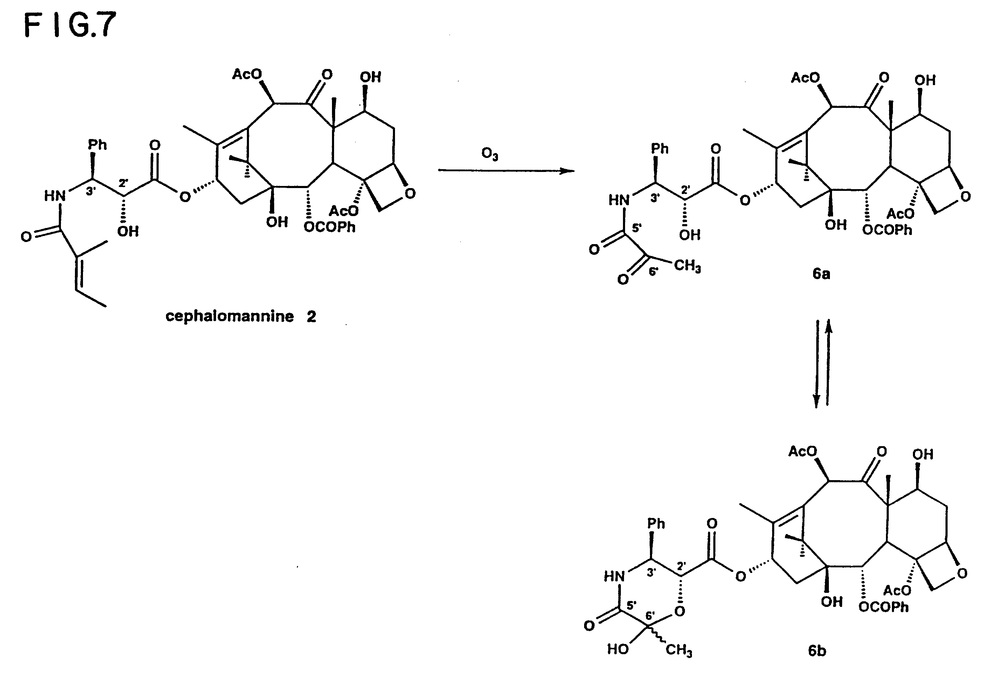

The first step of the process of the present invention, ozonolysis of cephalomannine and related compounds in the presence of taxol and other taxanes, is efficient. For very pure mixtures of cephalomannine and taxol, between, but not limited to, 1 to 10 molar equivalents at room temperature of ozone are required for complete oxidation of the cephalomannine which does not result in significant loss of any of the taxol. For impure mixtures of taxol, cephalomannine, and other compounds the length of ozone treatment is dependent upon the concentration of impurities that react with ozone. The functional group transformation for cephalomannine from the ozonolysis oxidation step is shown in Figure 7.

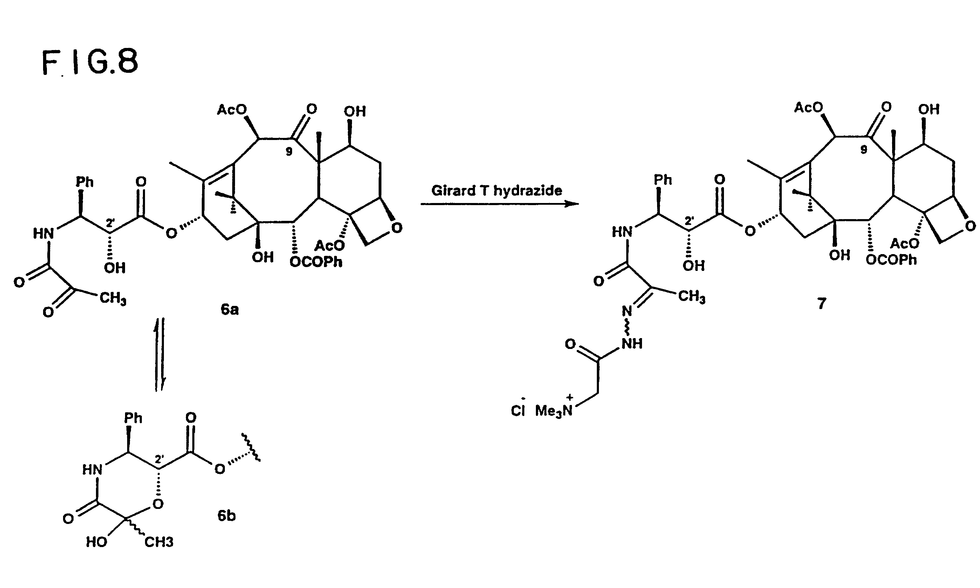

The second step of the process of the present invention, conversion of oxidized cephalomannine and related compounds to Girard hydrazones in the presence of taxol and other taxanes, is also efficient. For very pure mixtures of oxidized cephalomannine and taxol, between, but not limited to, 1 to 10 molar equivalents at 50°C of Girard reagent are required for complete conversion of the oxidized cephalomannine to the hydrazone derivative. The conversion does not result in significant loss of any of the taxol. The hydrazone formation is very selective for certain ketones in the taxane series of compounds. For example, the ketone at C-9 (see Figure 1 and Figure 8) is not reactive in the process of the present invention. It is apparently too hindered. The amount of Girard reagent needed to fully convert the oxidized cephalomannine depends upon the concentration of the other reactive compounds (impurities). The functional group transformation for oxidized (from ozone) cephalomannine for the hydrazone formation is shown in Figure 8.

The third step of the process of the present invention, separation of the hydrazones of oxidized cephalomannine and related compounds from taxol and other taxanes, is also efficient. For very pure mixtures of taxol and the Girard hydrazone of oxidized cephalomannine, selective precipitation or liquid/liquid extraction leads to isolation of taxol with high recovery. For impure mixtures of taxol, Girard hydrazones of oxidized cephalomannine and related compounds, the separation works equally well, but larger volumes of solvent and processes such as continuous liquid/liquid extraction may be required for high recovery of taxol.

The three steps of the process described herein are mild. The length of treatment time for complete oxidation of cephalomannine and related compounds with no significant loss of taxol to oxidation can be carefully maintained. Once again, the length of ozone treatment depends upon the concentration of impurities. The length of treatment time for complete conversion of oxidized cephalomannine and related compounds to Girard hydrazones with no significant loss of taxol can be carefully maintained. The amount of Girard reagent added depends upon the concentration of reactive impurities. The separation of the water soluble Girard hydrazones of cephalomannine and related compounds from taxol and other taxanes using liquid/liquid extraction or selective precipitation presents an extremely mild alternative to chromatography.

The separation process of this invention does not depend upon the ratio of cephalomannine and related compounds to taxol and other taxanes. For example, the ratio of cephalomannine to taxol in the crude extracts of Taxus brevifolia, as determined by High Pressure Liquid Chromatography -- HPLC (with the UV detector set at 227 nm), is approximately 0.2. Other types of biomass have variable ratios of taxol and cephalomannine. The process of the invention has been effective for mixtures of the two compounds at or below the ratio of the crude extract of Taxus brevifolia bark, and at ratios up to 7.35 (cephalomannine/taxol).

The oxidation via ozone exhibits many advantages for purification purposes over oxidation methods employing other chemical oxidants (e.g., hydrogen peroxide, perchloric acid, sodium periodate, potassium permanganate, sodium hypochlorite, osmium tetroxide and peracetic acid). Oxidation using ozone is more rapid, more selective for cephalomannine in the presence of taxol, more complete for short reaction times (minutes), and less toxic than the other listed oxidants. In addition, ozone oxidation is more amenable to treatment of very crude mixtures of taxol and other unreactive compounds and cephalomannine and related compounds than are other oxidants. Also, very important in the commercial purification of taxol is the fact that ozone can be introduced at an early point in the purification process, whereas this is not possible when using osmium tetroxide.

The overall separation process of this invention exhibits many other advantages over alternative methods that require chromatography. Chromatography (normal or reverse phase) is very expensive for a commercial process. The equipment required is highly specialized and expensive. The solid phase material is expensive, and after use it requires cleaning and/or disposal. Chromatographic processes require eluent fractionation which is time consuming and involves fraction analysis. Solvent systems and solvent recovery are often more complex than with more homogenous solvents and recycle streams such as those from a liquid/liquid extractive process, for example. In general, a process that does not require chromatography is more efficient and consequently less expensive than a process that requires chromatography.

In regards to purification, the process presented herein includes: selective ozone oxidation, conversion of oxidized taxanes to water soluble hydrazones, and separation of the hydrazones from other compounds, provides better overall selectivity and usefulness than is exhibited by the Kingston process while avoiding the expense and toxicity problems.

Other advantages of the process of the invention will be apparent from the detailed description section.

### Brief Description of Drawings

The method is described in more detail hereinafter with reference to the accompanying figures:

FIGURE 1 compares the structure of taxol and cephalomannine.

FIGURE 2 shows the reaction of cephalomannine with osmium tetroxide in the presence of taxol.

FIGURE 3 shows a flow diagram for separation of taxol and cephalomannine using osmium tetroxide and silica gel chromatography.

FIGURE 4 shows a flow diagram for separation of taxol and cephalomannine using ozone, followed by treatment with Girard hydrazide and a separation method.

FIGURE 5 shows the structure of the Girard T and Girard P reagents.

FIGURE 6 shows the functional group selectivity of ozone with different taxanes.

FIGURE 7 shows the reaction of cephalomannine and ozone.

FIGURE 8 shows the reaction of oxidized cephalomannine (from ozonolysis) and Girard T hydrazide.

FIGURE 9 shows a comparison of chemical oxidation by ozone and osmium tetroxide.

### Best Mode for Carrying Out the Invention

The present invention relates to the high yield selective process for the separation of cephalomannine and related compounds from taxol and other unreactive compounds in unpurified, partially purified, and purified mixtures. The separation methods of the present invention do not require normal phase or reverse phase chromatography. The Kingston process does require chromatography for the separation of taxol and cephalomannine. Chromatography is much more expensive and inefficient than the methods described in the present invention, especially on an industrial scale. These and other advantages of the process of the present invention over the Kingston process are described in detail below.

The Kingston process referred to above was run on purified and partially purified taxane mixtures that contained cephalomannine and taxol (see Figure 2). The partially purified mixture referred to was calculated to be 80% by weight for the taxol plus cephalomannine. No reference was made to the usefulness of their process for mixtures with purities below the 80% by weight level. Therefore, the Kingston process has not been evaluated for efficacy with a starting material that is very impure, for example, mixtures that contains less than 1% taxol by weight and less than 1% cephalomannine by weight. The Kingston process, and the process of the present invention are summarized in Figure 3 and Figure 4, respectively.

In our laboratories, the Kingston process was repeated using purified cephalomannine (90% cephalomannine by weight, no taxol) to confirm the usefulness of their process for high purity mixtures of taxanes. The experiment was run on 214 mg of cephalomannine (no taxol) using Kingston's reported method. The isolated product, 114 mg of cephalomannine-diol (57% yield), was characterized by spectroscopic methods and the data matched well with Kingston's reported data. The Kingston process was then performed on a dried, unpurified 505 mg sample of taxanes that contained 0.21% taxol by weight and 0.058% cephalomannine by weight (as determined by HPLC). Several portions of excess osmium tetroxide were required to fully oxidize the cephalomannine in the crude mixture. A total of approximately 2000 molar equivalents of osmium tetroxide relative to cephalomannine were added. The reaction required eight days and the resulting crude mixture was very thick and difficult to manipulate. No separation of the oxidized cephalomannine and taxol was possible due to the very difficult manipulation of the mixture.

The numerous negative features of the Kingston stoichiometric process for selective oxidation of cephalomannine in the presence of taxol when applied to very impure mixtures of taxanes include: the very long reaction time required for complete oxidation of cephalomannine; the very high ratio of osmium tetroxide to cephalomannine required for complete oxidation (the process is not economical - the osmium tetroxide reagent is very expensive - $76/gram from one common commercial supplier in 1993); the extremely careful handling required for the toxic osmium tetroxide reagent and the toxic waste associated with the process; and the difficulties associated with manipulation of very thick reaction mixtures due to the high concentration of toxic transition metal and transition metal salts in the mixture. Chromatography of these thick reaction mixtures, an essential step for the Kingston process, is very difficult.

The Kingston paper also describes a catalytic method for the oxidation of cephalomannine in the presence of taxol. The catalytic method is apparently provided by Kingston as a way to reduce the cost and toxicity problems associated with osmium tetroxide. The catalytic process was repeated using purified cephalomannine (90% cephalomannine by weight, no taxol) to confirm the usefulness of this process for purified mixtures of taxanes. The experiment was performed at 0°C on 5.9 mg of cephalomannine (90% by wt.) in acetone solvent to yield a mixture that contained the expected diol as determined by HPLC comparison with a standard of the diol that was prepared via the stoichiometric route. The Kingston process was then performed on a dried 1.4 g sample of impure taxanes that contained 0.21% taxol by weight and 0.058% cephalomannine by weight (as determined by HPLC). The taxane sample was not completely soluble in the acetone solvent system, even with sonication. Addition of the catalyst system followed by stirring resulted in no decrease in the amount of cephalomannine in the mixture even after several days reaction time. Therefore, the negative feature of the process for catalytic osmium tetroxide oxidation of cephalomannine in the presence of taxol as described by Kingston, is that it does not work well for less pure mixtures of taxanes.

The preferred embodiment of the present invention is a three step process. The first step includes contacting a mixture of taxanes containing cephalomannine and taxol with ozone in stoichiometric quantities (see Figures 4 and 7). The second step involves selectively converting the oxidized cephalomannine and other oxidized related compounds to water soluble hydrazones in the presence of taxol. The preferred water soluble hydrazones are Girard T or Girard P hydrazones. The third step includes separation of the water soluble oxidized taxane hydrazones from taxol and other unreactive compounds by selective precipitation, liquid/liquid extraction or other methods.

The present invention is effective for the separation of susceptible taxanes as very pure single compounds or in very pure mixtures. Samples of pure cephalomannine or a 50:50 mixture of pure taxol and pure cephalomannine, work well, for example. In addition, the present invention is also effective for the separation of susceptible taxanes in very crude mixtures of taxanes. The crude extracts of biomass also work well, for example. The Kingston process has only been demonstrated as useful for purified or partially purified mixtures of taxol and cephalomannine at greater than or equal to 80% purity by weight. It has been demonstrated herein that the Kingston process is not useful for very impure mixtures of taxanes. The usefulness of the present invention for the selective oxidation and separation from taxol of samples of susceptible taxanes at a wide range of purities without using chromatography as a separation step provides a significantly improved commercial process when compared to the process developed by Kingston.

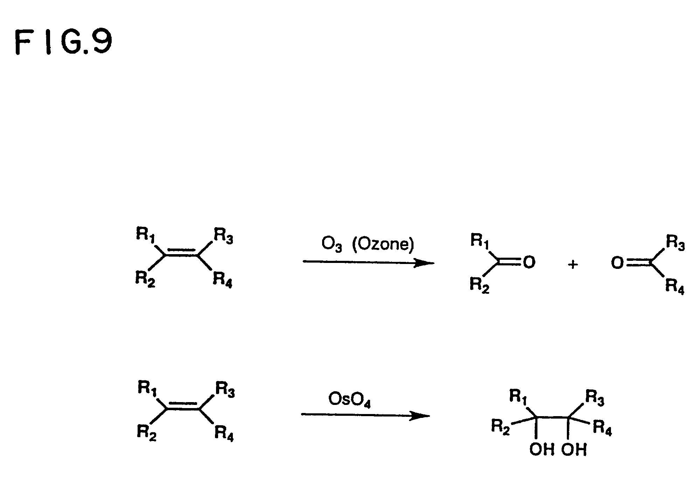

The first step of the process of the present invention, oxidation of purified cephalomannine by treatment with ozone, was performed on cephalomannine that was 90% pure by weight. Treatment of a solution of cephalomannine (2.4 g) in methylene chloride with 1.4 equivalents of ozone results in a quantitative yield of the desired compound which exists as the open and closed ring forms hereafter called **6a/6b**. The predominant form in non-alcoholic solvents is **6a** (see Figure 7). The compound mixture **6a/6b** has been fully characterized by spectroscopy and other methods. Figure 9 highlights the difference in products formed with the present invention versus the Kingston process. Ozone results in the oxidative cleavage of the olefin bond, whereas osmium tetroxide results in the dihydroxylation of the olefin bond. No olefin bond cleavage occurs with the osmium tetroxide process. Structures **3** and **6a/6b** show the different products formed by the two different processes. Structures **6a/6b** have two less carbons than **2** or **3** due to the oxidative cleavage. The experiment described above demonstrates the efficiency and unique nature of the oxidation part of the process described herein. The oxidation product of cephalomannine, **6a/6b,** has been fully characterized as the α-keto-amide derivative. An accessible carbonyl group (ketone or aldehyde) is crucial for the formation of the water soluble Girard hydrazone, the second step in the separation process. The diol formed by the Kingston process is not useful for the formation of a water soluble Girard hydrazone.

The process as a whole was performed on a mixture of pure cephalomannine and taxol. Cephalomannine (>95%) and taxol (>98%) were combined to make a 1:1 mixture (weight/weight) and dissolved in a suitable solvent, such as methylene chloride. The mixture was treated with 3 equivalents of ozone at -78°C. The oxidized mixture solution was concentrated and to it was added 1.5 equivalents of Girard T hydrazone and acetic acid for solvent. The solution was heated for 2 hours at 50°C and then evaporated to dryness. The residue was dissolved in methanol with some heating and water was carefully added to precipitate out the taxol selectively, leaving the water soluble Girard T hydrazone of oxidized cephalomannine in solution. The solid taxol was filtered out and recrystallized yielding 67.7% of the original taxol. This process has not been optimized but full details are given in Example VI. An alternative method (liquid/liquid extraction) for separation of the hydrazone and taxol is described in Example VII. It should be noted that the ozonolysis of the mixture of taxol and cephalomannine can be monitored by HPLC regardless of the purity of the starting mixture.

The process is not limited in the type of hydrazide or hydrazine that is suitable. The Girard P hydrazide is suitable for this process as well as the Girard T hydrazide. In addition, other water soluble hydrazines may work as well as the Girard hydrazides for the formation of water soluble hydrazones of the oxidized cephalomannine. The requirements for the water soluble hydrazide reagents for the process described herein include: high water solubility of the hydrazide; high reactivity with the pyruvamide ketone group of the oxidized cephalomannine; no reactivity with the C-9 ketone of taxol; high water solubility of the hydrazone(s) of the oxidized cephalomannine and related compounds; and stability of the hydrazone to the formation and manipulation steps for the separation process. In a more broad sense, hydrazide based materials that form hydrazones that have different solubility than unreactive compounds in the starting material, will also work. Reactive hydrazides bound to polymer supports, for example, should work well.

For very impure mixtures of cephalomannine and taxol containing ratios of 0.1 to 1.0 for cephalomannine/taxol (purity of taxol is between 0.1 and 1% by weight), the process is basically the same as for the purified mixtures. However, the process requires longer ozone treatment times and more equivalents of the Girard reagent for effective conversion. For example, dissolution of a mixture of dried biomass extract that contains taxol and cephalomannine in acetic acid, is followed by treatment with ozone (generally more than 50 molar equivalents relative to cephalomannine). The extent of cephalomannine oxidation can be carefully monitored by HPLC analysis of aliquots of the reaction mixture (see the Examples section). The solution is then purged with an inert gas such as argon or nitrogen. The Girard reagent is added (generally, more than 30 molar equivalents relative to the cephalomannine), and the mixture is heated to approximately 50°C for between one and twenty hours. The acetic acid solvent is then removed under vacuum. The separation of the hydrazones from the rest of the mixture (which contains taxol) can be effected using selective precipitation or liquid/liquid extraction. Normal or reverse phase chromatography may be used if it is advantageous, however, it is not mandatory for this separation process. Other separation processes that take advantage of the differing solubilities and differing polarities of the water soluble hydrazones versus taxol may be useful. Liquid/liquid extraction seems to work well for the very impure mixtures. For example, the residue is dissolved in a ternary mixture of ethyl acetate, water, and methanol (10:2:1). Other solvent systems may work as well. The resulting organic and aqueous phases are separated and the organic phase (which contains the taxol) is washed with water to remove residual aqueous impurities. The HPLC assay of the dried residue from the organic phase shows the recovery of taxol is excellent (>96% in all examples), and the purity of the taxol is generally increased by more than 100%. The mixture can be carried on to further purification. The cephalomannine, however, has been completely removed, thus alleviating one of the more difficult and expensive steps in the purification of taxol from extracts of biomass that also contains cephalomannine.

For partially purified mixtures of cephalomannine and taxol containing ratios of cephalomannine/taxol of 0.05 to 7.35 (purity of taxol or cephalomannine is between 1 and 5% by weight) the process is similar to that described directly above. The mixture of cephalomannine and taxol (a dry powder with purities ranging from 1% taxol to 99% taxol) is dissolved in an organic solvent (for example, acetic acid, methyl alcohol or methylene chloride), after which ozone is bubbled through the solution until the cephalomannine is completely oxidized. The reaction can be monitored by HPLC analysis as described in the Examples section. After the oxidation is finished, the reaction mixture is purged with argon or nitrogen gas to remove excess ozone. In all cases the cephalomannine and related compounds are oxidized with high recovery of taxol. The mixtures are then treated with the appropriate amount of Girard T or P hydrazide to effectively convert all of the oxidized cephalomannine and other susceptible oxidized taxanes to Girard T or P hydrazones. The separation of the hydrazones and unreacted hydrazide from the rest of the mixture (which contains taxol) can be effected using the methods outlined above.

The number of molar equivalents of ozone added is dependent upon the purity of the mixture (wt% of taxol and cephalomannine), and the concentration of cephalomannine and other compounds that react with ozone. Generally, less pure mixtures of taxanes require more molar equivalents (relative to cephalomannine) of ozone to completely oxidize the cephalomannine, than pure mixtures of taxanes. The less pure mixtures of taxanes contain several compounds that compete with the cephalomannine for the available ozone introduced into the mixture. See, for example, Figure 6, showing taxusin and brevifoliol, compounds which have olefin functional groups (outlined with the dotted line) that react with ozone.

The appropriate amount of Girard T or P hydrazide to use to convert the oxidized taxanes to hydrazones is based on an initial assay for cephalomannine and other taxanes in the starting mixture. Similar to the ozone oxidation step, less pure mixtures of taxanes require more molar equivalents (relative to cephalomannine) of Girard hydrazide reagent to completely form the hydrazone of the oxidized cephalomannine, than pure mixtures of taxanes. The hydrazone formation step is similar to the ozone oxidation step in that the conversion of the oxidized taxanes to hydrazones can be followed by HPLC. However, the hydrazones are not mobile in the analytical chromatography method used (see Example section). Consequently, observation that a peak is missing when comparing chromatograms of the starting oxidized mixture and the hydrazide/hydrazone mixture is a good indication that conversion of the oxidized compound to the hydrazone is complete.

Solvents that may be used for the ozone oxidation process include saturated hydrocarbons, halogenated solvents, alcohols, ethers, aldehydes, ketones, organic acids, esters, water, and mixtures thereof. Methylene chloride, chloroform, methyl alcohol, and acetic acid or mixtures thereof are the preferred solvents for the oxidation process.

For the more pure mixtures of cephalomannine and taxol (i.e., greater than 5% by weight of either cephalomannine or taxol) solvent may be added to dried solids to make solutions. The solutions are then treated with ozone for the appropriate time by monitoring the progress of the reaction by HPLC for the disappearance of the targeted compounds.

The hydrazone formation is effective in halogenated solvents, alcohols, ethers, organic acids, esters, water, and mixtures thereof. The preferred solvent is acetic acid.

Solvents effective for the selective precipitation include solvents generally miscible with water. Useful solvents include but are not limited to alcohols, ethers, aldehydes, ketones, organic acids, esters and mixtures thereof. The preferred solvent system is methanol/water.

Solvents effective for the liquid/liquid extraction include solvents generally immiscible with water, yet taxol has high solubility in these solvents. Useful solvents include but are not limited to saturated and unsaturated hydrocarbons, halogenated solvents, alcohols, ethers, aldehydes, ketones, esters and mixtures thereof. The preferred solvent systems are ethyl acetate/water or methylene chloride/water.

The oxidation process is effective over a wide range of temperatures. Temperatures that were investigated include but are not limited to -100°C to 50°C. The hydrazone formation is effective at temperatures at or above room temperature but below 100°C.

Ozone is available, for example, from an ozone generator capable of producing at least 8 grams of ozone an hour (0.42 pounds/day) at no less than 1% weight concentration in clean, dry air (-60° F. dewpoint) when operated with 115 volt, 60 Hertz current and 8 psig pressure; or at least 16 grams of ozone an hour (0.85 pounds/day) at no less than 2% weight concentration in pure, clean, dry oxygen (-60° F. dewpoint) when operated with 115 volt, 60 Hertz current and 8 psig pressure. The concentration of ozone in the oxygen stream can be determined by a variety of methods. Titrametric analysis of an acidic potassium iodide solution after specific treatment times with ozone is a preferred method. The use of sudan red 7B (a dye) has been reported for following ozonolysis reactions. In addition, calibrated ozone meters are available from certain manufacturers. The ozone may also be delivered to the reaction mixture as a solution of ozone in solvent.

The Girard reagents are available from several manufacturers. A review of the chemistry of this class of compounds has been provided by Wheeler, Chemical Reviews, Vol. 62, pp 205-221, 1962, incorporated herein by reference.

The use of ozone in an oxidation process with a fairly sensitive natural product such as taxol would not be expected to leave the taxol unaffected. This is especially true considering that taxol, cephalomannine, taxusin, and brevifoliol all have an olefin functional group in the A ring (see the arrows in Figure 6) which might be expected to react with ozone. In addition, taxol and other taxanes contain many functional groups that are susceptible to oxidation, such as tertiary hydrogen atoms, aryl rings, and ethers. The high yield and selective ozone oxidation of the tiglate tail portion of the cephalomannine side chain in unpurified, partially purified, and purified mixtures of taxanes, under a variety of conditions is not obvious to one skilled in the art.

The formation of the Girard T or P hydrazones of the oxidized taxanes, particularly oxidized cephalomannine, is selective and efficient. The reactions can be monitored by HPLC for completion. The products formed have high water solubility. The separation of the water soluble hydrazones of oxidized cephalomannine and taxol is selective and efficient also. Even though the process presented herein requires an additional step when compared to the Kingston process, the advantages of no chromatography and the useful industrial application of the process to mixtures containing taxol at a wide range of purities renders the extra step acceptable. Examples VI, VII, and VIII highlight the positive features of the three steps of the present invention when compared to Examples III and IV. It is important to note that other water soluble hydrazones may work as well as the Girard hydrazones of the oxidized compounds, for the separation of taxol and other unreactive compounds from cephalomannine and related compounds, in this process. General structures for water soluble hydrazides and hydrazines that may be useful for the present process are shown in Figure 5.

In the process of this invention, taxol is not significantly degraded by ozone, and the selective oxidation of cephalomannine and related compounds in the presence of taxol and other unreactive compounds using ozone relies upon the functional group differences between cephalomannine along with related compounds, and taxol. It is important to note, however, that taxol will degrade to several unidentified compounds by improper exposure to a large excess of ozone or from improper exposure to a slight excess for an extended period. The degradation of taxol by ozone can be avoided if the reaction is monitored carefully by TLC or HPLC. The following examples provide procedures for carrying out the selective ozone oxidation of samples containing taxol and cephalomannine without significant degradation of taxol.

The positive features of the present invention when compared to the Kingston process include: very fast reaction (oxidation) times; easily controlled addition of oxidizing reagent; a process that is flexible enough to selectively and efficiently oxidize cephalomannine and related compounds in mixtures containing taxol at a wide range of purities (purity levels tested include mixtures containing cephalomannine and taxol at less than 0.3% by weight to mixtures that contain cephalomannine and taxol at greater than 95% by weight in a 1:1 mixture); inexpensive and easily handled reagents and reaction mixtures; efficient and selective formation of water soluble hydrazone derivatives of the oxidized taxanes; inexpensive and efficient methods of separation of the water soluble hydrazones of oxidized taxanes and taxol; and very little toxic waste associated with the reaction and separation.

### Examples

The following examples provide specific processes for the selective oxidation of cephalomannine and other susceptible compounds in unpurified, partially purified or purified samples of taxanes. These selective oxidations work equally well for samples that contain ozone susceptible compounds with or without taxol or cephalomannine. The following examples also provide a method for separating the oxidized compounds, specifically oxidized cephalomannine from taxol and other unreactive taxanes. All scientific and technical terms have the meanings as understood by one with ordinary skill in the art. The term "biomass" as used herein includes Taxus species and cell culture of Taxus species. The impure mixtures, ie., crude biomass extracts can be obtained, for example, by methods described in a recent publication (Rao, Koppaka V., "Method for the Isolation and Purification of Taxane Derivatives", International Publication Number, WO 92/07842, May 14, 1992), incorporated herein by reference.

All solvents and reagents employed were used as received from the manufacturer except pyridine which was distilled prior to use. Reactions were monitored by thin-layer chromatography ("TLC") using 0.20 mm. E. M. Industries Silica Gel 60 (aluminum support) silica gel plates. Reactions were also monitored by high pressure liquid chromatography ("HPLC"). Aliquots of crude reaction mixtures for HPLC analysis were removed from the reaction vessel with a 3 µl micro-pipette and diluted to 200 µl in an HPLC sample vial (with insert). The HPLC system consists of a model L-6200 pump, Model AS-4000 or L-3000 UV/VIS/DAD detector (Hitachi Instruments, Inc.). The system was equipped with an NEC 286 computer with 40M hard drive and Lab Manager HPLC software (Hitachi Instruments, Inc.). HPLC columns used included a 4.6 mm. X 250 mm. phenyl column, packed with 5 µm diphenyl material (Supelco, Inc.); a 4.6 mm. X 250 mm., 5 µm, 60 angstrom pentafluorophenyl (PFP) column (ES Industries); and a 4.6 mm. X 250 mm. phenyl guard column (Jones Chromatography). The ozone generator used was a Polymetrics Laboratory Ozonator T-816. The ozone delivery concentration was 0.0046 mmole/second at settings of 100 volts, 60 Hz current, 3.0 psig pressure and a flow rate of 2 SLMP. The ozone flow was calibrated using the method described by the manufacturer. Silica gel for flash chromatography (230 to 400 mesh) was supplied by Scientific Products. A Bruker WP-270 and ACE-300, Varian Gemini 400, and a JEOL FX90Q Spectrometer were employed for ¹H and ¹³C NMR spectra with chemical shifts reported in ppm. relative to tetramethylsilane using residual non-deuterated NMR solvent for reference. Yields refer to chromatographically pure compounds and are not optimized. Purity of products were judged to be >90% on the basis of spectrophotometric homogeneity unless otherwise stated. Chromatographic purity as used herein refers to the HPLC normalized peak area percentage at 227 nm for a given component. Mass spectra were measured at M-Scan Inc. using a VG Analytical 2-SE high field mass spectrometer or a VG platform mass spectrometer using electrospray mode. Spectroscopic analyses were determined using an Analect Diamond-20 FTIR with an XAD-Plus microscope. The instrument was equipped with an ACR Advanced Logic Research 486 computer with 200M hard drive and an Analect FX80 software package.

### Example I (Demonstration of Prior Art)

**Procedure for oxidation of purified cephalomannine with osmium tetroxide (stoichiometric method).** OsO₄ in pyridine (0.020M, 24.5 ml, 2.1 equivalents) was added to a solution of cephalomannine (192 mg, 0.23 mmol) in dry pyridine (12.88 ml, 0.02M) and mixed for 19 hours at room temperature. The reaction was then quenched with 10% NaHSO₃ (43 ml) and mixed for 2.5 hours. The mixture was acidified with 3N HCl to pH 1 (paper) and extracted 3 times with EtOAc. The organic layer was washed twice with brine, dried over Na₂SO₄, and evaporated. The light green residue was chromatographed on silica gel (gradient EtOAc/hexane) giving 114 mg (57% yield) of a white solid (R_{f} = 0.41, 10:90 MeOH/CH₂Cl₂). The spectrometric analyses of the solid match with Kingston's reported values ("Modified Taxols, 7. A Method For The Separation Of Taxol And Cephalomannine", Journal of Natural Products, 55, 259-261, (1992)).

### Example II (Demonstration of Prior Art)

**Procedure for oxidation of purified cephalomannine with osmium tetroxide (catalytic method).** To 5.9 mg (0.007 mmol) of cephalomannine dissolved in acetone was added tetraethylammonium acetate (0.7 mg, 0.003 mmol, 0.43 equivalents). The mixture was cooled to 0°C and t-butyl hydroperoxide (1.7 µl, 2.54 equivalents of a 70% H₂O solution) and osmium tetroxide (2.5 µl, 0.029 equivalents of a 0.070 M solution in t-butyl alcohol) were added. The mixture was stirred for one hour at 0°C and then 1.5 hours at room temperature. A sample was analyzed by HPLC and the reaction appeared to be finished. The HPLC and TLC data indicate a very similar product mixture was formed by the catalytic method as by the stoichiometric method.

### Example III

**Oxidation of impure cephalcmannine/taxol with osmium tetroxide (stoichiometric method).** Dried natural product extract (505.3 mg) containing cephalomannine (0.3 mg, 0.058% by weight) was dissolved in dry pyridine (1.3 ml). To the solution was added in portions OsO₄ in pyridine (0.077 M) over a seven day period at room temperature until no cephalomannine remained as shown by HPLC. It took between 1,176 and 1,990 equivalents of OsO₄ (5.345 ml and 9.045 ml) to completely oxidize the available cephalomannine.

### Example IV

### Oxidation of impure cephalomannine/taxol with osmium tetroxide (catalytic method).

A suspension of dried, ground biomass extract (1.40 g) containing 0.81 mg cephalomannine (0.058% by weight) was prepared in acetone (1.5 ml originally), and 3 x 0.5 ml portions were added to facilitate mixing. The initially liquid/granular mixture turned into a liquid/gummy tar within ten minutes. The liquid was sampled for HPLC analysis, thus confirming the presence of cephalomannine. Tetraethylammonium acetate (0.08 mg, 0.32 equivalents) was added and the mixture was cooled to 0°C. To this mixture was added t-butyl hydroperoxide (0.3 µl, 2.28 equivalents of a 70% H₂O solution) followed by addition of osmium tetroxide (0.3 µl, 0.025 equivalents of a 0.79 M solution). The mixture was stirred for 1.25 hours at 0°C, and then 2 hours at room temperature. At this point, no oxidation of cephalomannine was observed when a small aliquot was removed from the mixture and analyzed by HPLC. Additional aliquots were removed after 17.75 and 25.75 hours of total reaction time. The HPLC analysis of these samples indicated no decrease in the amount of cephalomannine or increase in the amount of expected diol.

### Example V

### Procedure for the oxidation of pure cephalomannine with ozone.

Cephalomannine **2** (90% by weight, total weight: 2.702 g; cephalomannine: 2.432 g, 29.24 mmol) in CH₂Cl₂ (65 ml, 0.45 M) at -60 to -75°C was treated with ozone (39.744 mmol, 0.0046 mmol/s, 1.4 equivalents) for 184 minutes. The solution was then purged with argon at room temperature for 5 minutes following evaporation. Analysis by HPLC indicated the reaction was complete, and NMR showed the equilibrium mixture of **6a** and **6b**. Resonances for the major compound are listed. ¹H NMR (90 MHz, CDCl₃) 1.11 (s, 3H), 1.21 (s, 3H), 1.29 - 1.58 (m, 2H), 1.64 (s, 3H), 1.78 (s, 3H), 1.89 - 2.16 (m, 3H), 2.20 (s, 3H), 2.31 (s, 3H), 2.24 (s, 3H), 2.40 - 2.72 (m, 1H), 3.75 (d, J = 6.8 Hz, 2H), 3.98 - 4.47 (m, 3H), 4.64 (m, 1H), 4.89 (d, J = 8.5 Hz, 1H), 5.13 - 5.68 (m, 2H), 5.98 - 6.20 (m, 1H), 6.25 (s, 1H), 7.27 - 7.73 (m, 8H), 7.85 (d, J = 9.4 Hz, 1H), 8.07 (d, J = 7.7 Hz, 2H). ¹³C NMR (12 MHz, CDCl₃) 9.54, 14.65, 20.76, 21.65, 22.51, 24.37, 26.79, 35.62, 35.62, 43.12, 45.72, 55.00, 58.48, 72.04, 72.04, 73.31, 74.96, 75.58, 76.44, 79.00, 81.16, 84.32, 126.97, 126.97, 128.60, 128.60, 128.60, 128.89, 128.89, 129.16, 130.11, 130.11, 133.24, 133.65, 137.14, 141.68, 159.70, 166.88, 170.30, 171.07, 171.93, 195.94, 203.55. The diagnostic signals in the ¹³C NMR spectrum for the minor isomer in CDCl₃ (carbon 6' of the two diastereomers of **6b**) are 102.52 and 105.35 ppm. The diagnostic signals in CD₃OD of **6ab**, including possible solvent addition (CD₃OD) to both **6a** and **6b** are 97.9 and 197.2 ppm. FTIR (neat, cm⁻¹) 981.6 (m), 1025.9 (m), 1070.3 (m), 1108.9 (m), 1178.3 (m), 1241.9 (s), 1373.1 (m), 1724.0 (s), 2900.4 (w), 2940.9 (w), 3064.3 (w), 3413.4 (m), 3490.5 (m). The melting point of an analytically pure sample (chrom purity >97%) was 162-167°C. Mass Spectrum (FAB, glycerol/thioglycerol matrix) m/z 821 (M + 1)⁺.

### Example VI

**Oxidation of pure cephalomannine/taxol with ozone and separation using Girard T reagent and selective precipitation.** A mixture of pure taxol (100.6 mg, 0.119 mmole) and cephalomannine (100.5 mg, 0.121 mmole) was dissolved in dry methylene chloride. The mixture was cooled to -78°C in a dry ice/acetone bath and 3 molar equivalents (compared to cephalomannine) of ozone was bubbled into the solution. After purging the solution with argon, the solvent was removed from the mixture. To the dried reaction mixture was added 1.5 molar equivalents (compared to the original molar amount of cephalomannine) of Girard T hydrazide reagent (30.8 mg). To dissolve the solids, enough acetic acid was added (880 microliters) to make a 0.14 M solution. The reaction mixture was heated to 50°C with stirring for 2 hours. The acetic acid was evaporated and 800 microliters of methanol (8 ml/1 g of solids) was added to dissolve the mixture. The solution was then heated to 50°C, and enough water (200 microliters) was added slowly to make a 25% H₂O/MeOH solution. The solution was allowed to cool slowly to room temperature and then it was placed in a freezer overnight. The following morning the solids (taxol) were isolated by vacuum filtration. After drying, the recovery of taxol was 79.6 mg (79.1%). The solids were recrystallized in MeOH/H₂O to yield 68.1 mg (67.7% recovery) . The chromatographic purity of the recovered taxol is 97.5%. The m.p. is 198 - 201°C; the m.p. for a taxol standard: 208 - 212°C. The ¹³C NMR data for the isolated taxol matched exactly with that of a standard sample.

### Example VII

### Oxidation of pure cephalomannine/taxol with ozone and separation using Girard T reagent and liquid/liquid extraction.

An equal mixture of pure cephalomannine (19.7 mg, 0.024 mmol) and pure taxol (19.7 mg, 0.023 mmol) were dissolved in 1.2 ml of acetic acid and treated with 2 molar equivalents of ozone (relative to cephalomannine). The reaction mixture was purged with argon and 5 molar equivalents (relative to cephalomannine) of the Girard T reagent (20.1 mg) was added and the solution was heated to 50°C. After heating for 2 hours, the solvent was removed under vacuum. The dried reaction mixture was dissolved in a minimal amount of ethyl acetate and water and the two phases were separated. The organic phase that contains the taxol was washed sequentially with saturated sodium bicarbonate and brine solutions. The total residue (19.1 mg) after evaporation was 95% taxol by chromatographic purity analysis with a 93% overall recovery.

### Example VIII

### Oxidation of impure cephalomannine/taxol with O₃ and separation using Girard T reagent and liquid/liquid extraction.

A crude methanol extract of *Taxus brevifolia* bark was assayed. The cephalomannine purity was calculated to be 0.04% and the taxol purity was calculated to be 0.20%. The dried crude extract (52.89 g) was dissolved in a minimum amount of acetic acid (350 ml) and was treated with 50 molar equivalents (relative to cephalomannine) of ozone. After the solution was purged with argon, 121.7 mg, (30 molar equivalents relative to cephalomannine) of the Girard T reagent was added and the solution was heated to 50° C in an oil bath. After mixing for 2 hours, the solution was removed from the heat and the acetic acid was removed under vacuum. The dried reaction mixture was then dissolved in ethyl acetate (500 ml), water (100 ml) and methanol (45 ml). The methanol was added to the solution to aid in the dissolution of the solids. The solution was rinsed into a separatory funnel and the two phases were separated. The organic phase containing the taxol was washed with water to remove residual aqueous phase impurities. Analysis of the organic phase by HPLC showed the recovery of taxol to be 97% with a 60% decrease of impurities. The organic phase was evaporated and assayed again by HPLC. The overall taxol purity was increased to 0.5% (from 0.2%) and cephalomannine and oxidized cephalomannine were completely removed from the mixture.

Other variants are possible without departing from the scope of this invention.

## Claims

1. A process for separating cephalomannine from a mixture containing cephalomannine and taxol in a solution, said process comprising the steps of:
(a) contacting said mixture with ozone thereby oxidizing cephalomannine to form oxidized cephalomannine;
(b) converting said oxidized cephalomannine to a water-soluble hydrazone; and
(c) separating said water-soluble hydrazone from said mixture,
wherein said mixture is prepared by dissolving a dried biomass extract into a solvent selected from the group consisting of hydrocarbons, halogenated solvents, alcohols, ethers, aldehydes, ketones, amines, organic acids, organic acid derivatives, esters, water and mixtures thereof.

2. The process of claim 1, wherein the ozone is bubbled through said mixture.

3. The process of claim 1, wherein the ozone is contained in a liquid which is added to said mixture.

4. The process of claim 1, wherein said water-soluble hydrazone is produced by contacting said mixture containing said oxidized cephalomannine with a water-soluble hydrazide or hydrazine.

5. The process of claim 4, wherein said water-soluble hydrazide is a Girard T or Girard P hydrazide having the formula

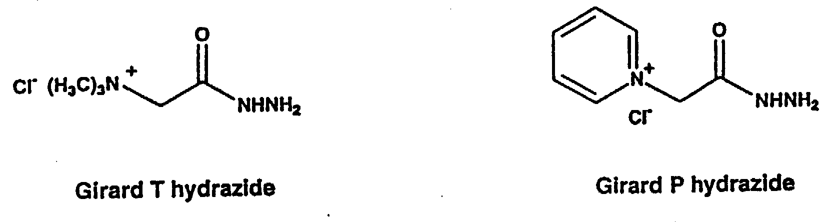

6. The process of claim 1, wherein said water-soluble hydrazone is separated from said mixture by means of selective precipitation, liquid/liquid extraction, or chromatography.

7. The process of claim 1, wherein said dried biomass extract is an extract of *Taxus brevifolia.*

8. A process for preparing a water-soluble hydrazone derivative of cephalomannine in a solution, comprising the steps of:
(a) oxidizing the cephalomannine by contacting the solution with ozone to form oxidized cephalomannine;
(b) contacting said oxidized cephalomannine in the solution with a water-soluble hydrazide to form a water-soluble hydrazone; and
(c) separating said water-soluble hydrazone from the solution.

9. A compound of the formula: 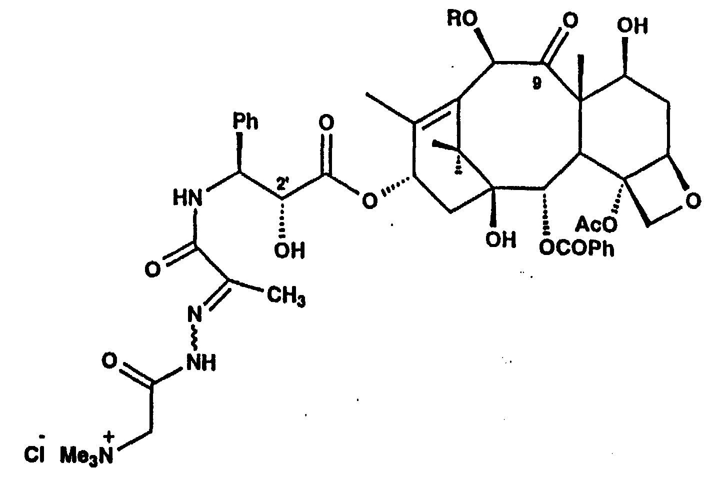 wherein R denotes an acetyl radical or hydroxyl radical.

10. A compound of the formula: 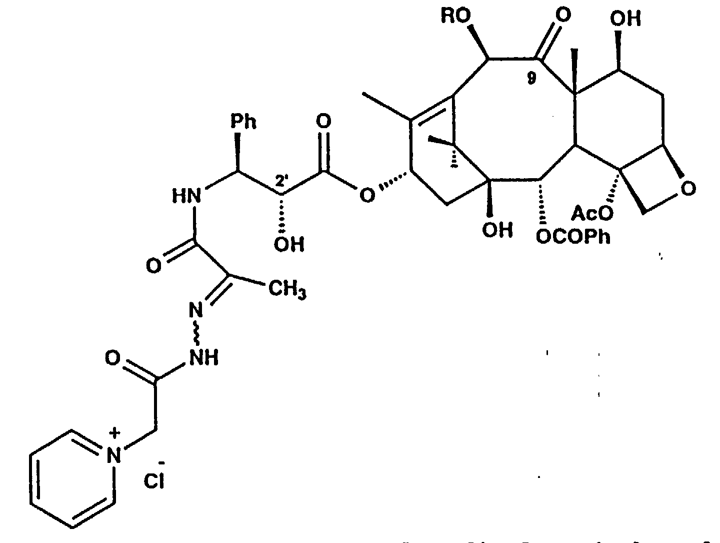 wherein R denotes an acetyl radical or hydroxyl radical.

## Patentansprüche

1. Verfahren zum Abtrennen von Cephalomannin aus einem Gemisch, enthaltend Cephalomannin und Taxol in einer Lösung, welches Verfahren die Schritte umfaßt:
(a) Kontaktieren des Gemisches mit Ozon, wodurch das Cephalomannin unter Erzeugung von oxidiertem Cephalomannin oxidiert wird;
(b) Überführen des oxidierten Cephalomannins in ein wasserlösliches Hydrazon; sowie
(c) Abtrennen des wasserlöslichen Hydrazons von diesem Gemisch, wobei das Gemisch aufbereitet wird durch Auflösen eines getrockneten Biomasse-Extraktes in einem Lösemittel, ausgewählt aus der Gruppe, bestehend aus Kohlenwasserstoffen, halogenierten Lösemitteln, Alkoholen, Ethern, Aldehyden, Ketonen, Aminen, organischen Säuren, Derivaten von organischen Säuren, Estern, Wasser und Mischungen davon.

2. Verfahren nach Anspruch 1, bei welchem das Ozon durch das Gemisch hindurchgeperlt wird.

3. Verfahren nach Anspruch 1, bei welchem das Ozon in einer Flüssigkeit enthalten ist, die dem Gemisch zugesetzt wird.

4. Verfahren nach Anspruch 1, bei welchem das wasserlösliche Hydrazon erzeugt wird, indem das Gemisch, das das oxidierte Cephalomannin enthält, mit einem wasserlöslichen Hydrazid oder Hydrazin kontaktiert wird.

5. Verfahren nach Anspruch 4, bei welchem das wasserlösliche Hydrazid ein Girard T-Hydrazid oder Girard-P-Hydrazid ist und die Formel hat:

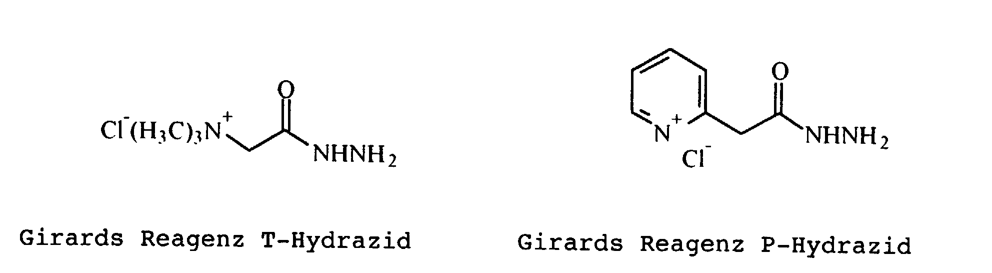

6. Verfahren nach Anspruch 1, bei welchem das wasserlösliche Hydrazon aus dem Gemisch durch selektives Ausfällen, Flüssig/Flüssig-Extraktion oder durch Chromatographie abgetrennt wird.

7. Verfahren nach Anspruch 1, bei welchem der getrocknete Biomasse-Extrakt ein Extrakt aus *Taxus brevifolia* ist.

8. Verfahren zum Herstellen eines wasserlöslichen Hydrazon-Derivats von Cephalomannin in einer Lösung, umfassend die Schritte:
(a) Oxidieren des Cephalomannins durch Kontaktieren der Lösung mit Ozon unter Erzeugung von oxidiertem Cephalomannin:
(b) Kontaktieren des oxidierten Cephalomannins in der Lösung mit einem wasserlöslichem Hydrazid, um ein wasserlösliches Hydrazon zu erzeugen; sowie
(c) Abtrennen des wasserlöslichen Hydrazons von der Lösung.

9. Verbindung der Formel: 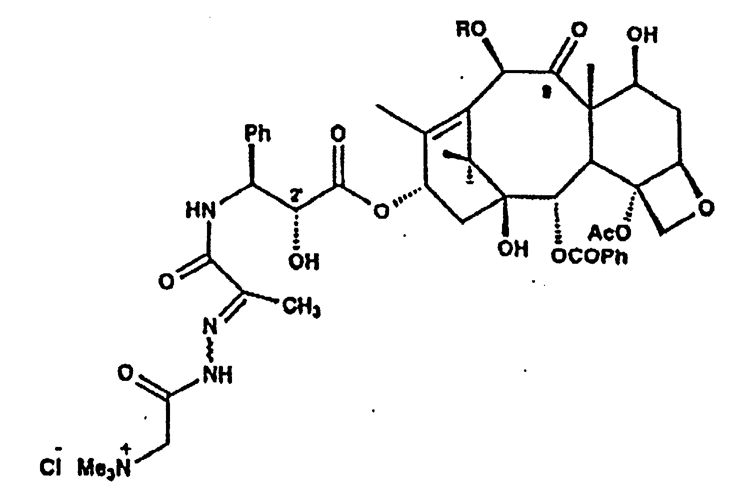 worin R einen Acetyl-Rest oder Hydroxyl-Rest bezeichnet.

10. Verbindung der Formel: 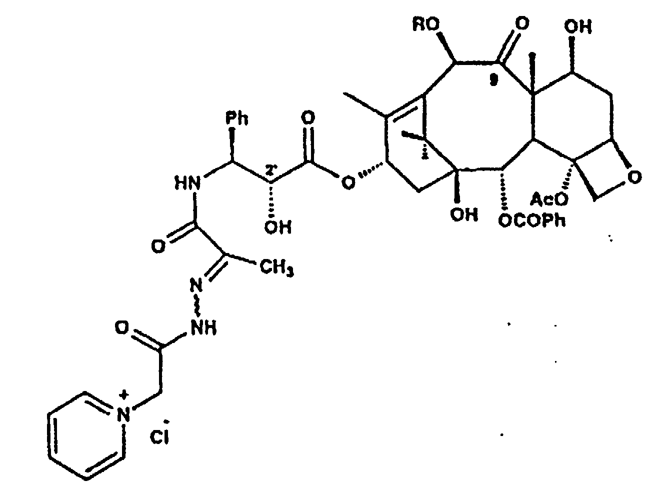 worin R einen Acetyl-Rest oder Hydroxyl-Rest bezeichnet.

## Revendications

1. Procédé pour séparer la céphalomannine d'un mélange contenant la céphalomannine et le taxol en solution, ledit procédé comprenant les étapes consistant à :
(a) mettre en contact ledit mélange avec de l'ozone afin d'oxyder la céphalomannine pour former la céphalomannine oxydée ;
(b) convertir ladite céphalomannine oxydée en hydrazone hydrosoluble ; et,
(c) séparer ladite hydrazone hydrosoluble dudit mélange, ledit mélange étant préparer par dissolution d'un extrait de biomasse séchée dans un solvant choisi parmi l'ensemble constitué par les hydrocarbures, les solvants halogénés, les alcools, les éthers, les aldéhydes, les cétones, les amines, les acides organiques, les dérivés d'acides organiques, les esters, l'eau et leurs mélanges.

2. Procédé suivant la revendication 1, dans lequel on fait barboter l'ozone dans ledit mélange.

3. Procédé suivant la revendication 1, dans lequel l'ozone est contenu dans un liquide qui est ajouté audit mélange.

4. Procédé suivant la revendication 1, dans lequel ladite hydrazone hydrosoluble est produite par mise en contact dudit mélange contenant ladite céphalomannine oxydée avec un hydrazide ou hydrazine hydrosoluble.

5. Procédé suivant la revendication 4, dans lequel ledit hydrazide hydrosoluble est un hydrazide T ou P de Girard de formule :

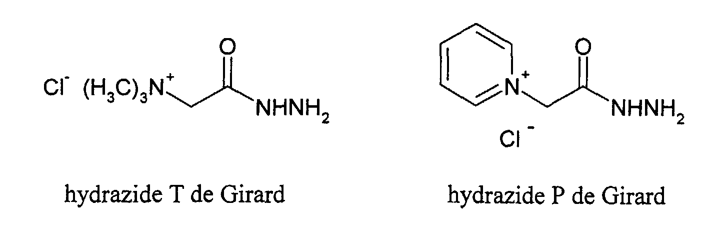

6. Procédé suivant la revendication 1, dans lequel ladite hydrazone hydrosoluble est séparée dudit mélange par précipitation, extraction liquide/liquide ou chromatographie sélective.

7. Procédé suivant la revendication 1, dans lequel ledit extrait de biomasse séchée est un extrait de *Taxus brevifolia.*

8. Procédé pour préparer une hydrazone hydrosoluble dérivant de céphalomannine en solution, ledit procédé comprenant les étapes consistant à :
(a) oxyder la céphalomannine par mise en contact de la solution avec de l'ozone pour former la céphalomannine oxydée ;
(b) mettre en contact ladite céphalomannine oxydée en solution avec un hydrazide hydrosoluble pour former une hydrazone hydrosoluble ; et,
(c) séparer ladite hydrazone hydrosoluble de la solution.

9. Composé de formule : 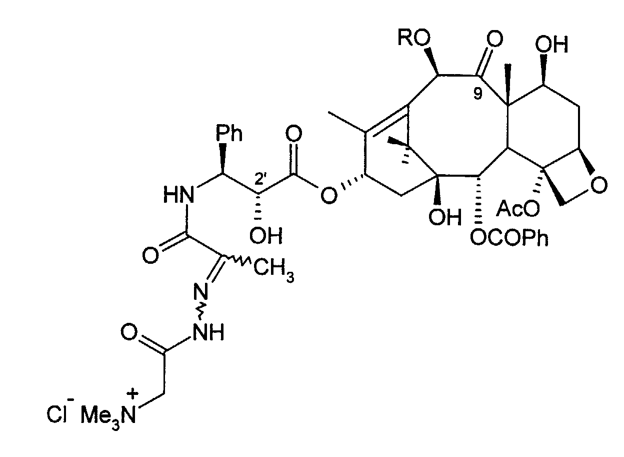 où R représente un radical acétyle ou hydroxyle.

10. Composé de formule : 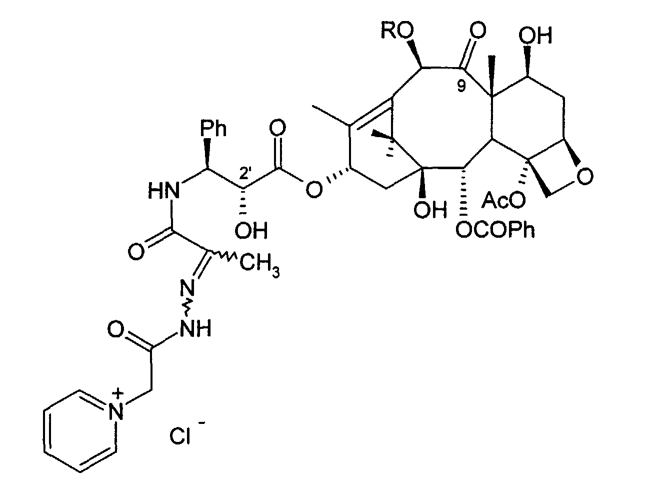 où R représente un radical acétyle ou hydroxyle.
